(19) Europäisches Patentamt / European Patent Office / Office européen des brevets

(11) **EP 4 187 312 A1**

(12) **EUROPEAN PATENT APPLICATION**
published in accordance with Art. 153(4) EPC

(43) Date of publication:
**31.05.2023 Bulletin 2023/22**

(21) Application number: **21851272.1**

(22) Date of filing: **29.07.2021**

(51) International Patent Classification (IPC):
**G02C 13/00** (2006.01)   **A61L 12/02** (2006.01)
**A61L 12/10** (2006.01)

(52) Cooperative Patent Classification (CPC):
**A61L 2/18; A61L 12/02; A61L 12/10; G02C 13/00;**
A61L 2101/00

(86) International application number:
**PCT/CN2021/109100**

(87) International publication number:
**WO 2022/022603 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020 CN 202010760135**

(71) Applicant: **Suzhou 3N Biological Technology Co., Ltd**
**Suzhou, Jiangsu 215000 (CN)**

(72) Inventor: **SUN, Bixia**
**Suzhou, Jiangsu 215000 (CN)**

(74) Representative: **Sun, Yiming**
**HUASUN Patent- und Rechtsanwälte**
**Friedrichstraße 33**
**80801 München (DE)**

(54) **CONTACT LENS CLEANER, AND PROTEIN REMOVAL AND STERILIZATION METHOD BY MEANS OF ELECTROPHORESIS DISSOCIATION**

(57)   A contact lens cleaner and a method for protein removal and sterilization by electrophoresis dissociation are disclosed. The contact lens cleaner comprises a lens cleaning tank (10), two electrophoresis dissociation probes (20) are arranged in the lens cleaning tank (10) opposite each other, and the electrophoresis dissociation probes (20) are electrically connected to a circuit power supply. The lens cleaning tank (10) is filled with an electrolyte solution; a lens to be cleaned is placed in the lens cleaning tank (10); and a switch is turned on, and the two electrophoresis dissociation probes (20) in the lens cleaning tank (10) form an anode and a cathode. There are lacrimal proteins on the surface of the lens to be cleaned, the lacrimal proteins are charged in the solution, and the charged lacrimal proteins move to the electrode opposite to the electrical property thereof in the cleaning tank (10) according to the phenomenon of electrophoresis. The electrolyte solution reacts chemically in the cleaning tank (10) to produce hypochlorite having a strong oxidizing property, and the hypochlorite can degrade the lacrimal proteins, and also can sterilize and disinfect.

Fig.1

EP 4 187 312 A1

**Description**

**Field of the Invention**

The present disclosure relates to the field of protein removal and sterilization of orthokeratology lens, and particularly relates to a protein removal sterilization method that can not only remove lacrimal protein on a surface of a contact lens, but also sterilize and disinfect by electrophoresis dissociation, and a contact lens cleaner for implementing protein removal and sterilization by electrophoresis dissociation.

**Background technique**

The problem of how to remove proteins from a corneal contact lens has troubled the industry for more than half a century, which has caused high attention of the eye and vision industry in various countries to the wearing safety of the contact lens. In China, because corneal infection caused by the corneal contact lens arise frequently, the contact lens was listed in type III medical apparatus and instruments in 2012 for high-risk management. The reason is that a material structure of the contact lens has a large number of invisible fiber oxygen permeation pores, eyes will secrete a large amount of tears all the time, and the tears contain a large amount of lacrimal proteins, which can extremely easily permeate into the fiber oxygen permeation pores to cause a reduced DK value of lenses (oxygen permeability) and symptoms such as corneal hypoxia and edema; and in serious cases, it may cause problems of corneal damage, bacterial infection, keratitis, even vision damage, etc.

In order to remove bacteria on the surface of the contact lens, a traditional cleaning method is to physically rub the lens with a care solution or soak the lens in a chemical active agent so as to achieve the purpose of protein removal. However, both domestic and foreign experimental data and user feedback have proved that the protein removal effect of such means is very small, and the lens is prone to being scratched and damaged by finger rubbing.

Therefore, it is urgently needed to provide a new technical solution to solve the above problems.

**SUMMARY OF THE INVENTION**

An objective of the present disclosure is to solve the problems in the prior art. In an aspect, a method for protein removal and sterilization by electrophoresis dissociation is provided. The technical solutions used are as follows.

A method for protein removal and sterilization by electrophoresis dissociation comprises: providing a contact lens cleaner comprising a lens cleaning tank having two electrophoresis dissociation probes arranged opposite each other therein; adding a lens to be cleaned and an electrolyte solution serving as a cleaning solution into the lens cleaning tank, the electrolyte solution comprising a solution containing $Cl^-$, and forming an anode and a cathode by the two electrophoresis dissociation probes under a circuit loop; enabling lacrimal proteins attached to a surface of the lens to be cleaned to be charged in the solution containing $Cl^-$, and enabling the charged lacrimal proteins to move towards a position of one electrode opposite to an electrical property thereof; enabling the $Cl^-$ in the electrolyte solution to move towards the anode, and to lose electrons and be oxidized into chlorine which is dissolved in the electrolyte solution to generate hypochlorite; and the hypochlorite reacting with the lacrimal proteins in an oxidation reaction in the lens cleaning tank to degrade the lacrimal proteins.

According to the technical solution, the hypochlorite is a strong oxidant, and undergoes a self-redox reaction in the electrolyte solution so as to be decomposed to generate hydrogen ions, chloride ions and oxygen; and the hypochlorite can absorb electrons of functional proteins on cell walls of microorganisms in the lens cleaning tank during decomposition, so that the functional proteins on the cell walls of the microorganisms are inactivated, and the microorganisms are killed; and/or, the hypochlorite absorbs electrons of functional proteins on shells of the microorganisms in the lens cleaning tank during decomposition, so that the functional proteins on the cell walls of the microorganisms are inactivated, and the microorganisms are killed; and/or, the hypochlorite permeates into cells of the microorganisms and undergoes an oxidation reaction with mycoprotein, nucleic acid and enzymes in the cells of the microorganisms, so that the microorganisms are killed.

In a further embodiment, the microorganisms comprise bacteria and viruses; the hypochlorite absorbs the electrons of the functional proteins on the cell walls of the bacteria during decomposition, and thus the bacteria are inactivated; and the hypochlorite absorbs the electrons of the functional protein on the shells of the viruses during decomposition, and thus the viruses are inactivated.

In a further embodiment, the hypochlorite reacting with the lacrimal proteins in an oxidation reaction in the lens cleaning tank to degrade the lacrimal proteins specifically comprises: the hypochlorite reacts with a peptide chain forming a lacrimal protein framework to generate chloral formamide; a peptide chain formed by chloral formamide is hydrolyzed in the solution; and peptide bonds in the peptide chain are broken and decomposed into small molecular protein and amino acid, and thus the lacrimal proteins are degraded; and/or the hypochlorite reacts with a side chain of the peptide chain forming the lacrimal protein framework; the side chain comprises a lysine side chain which reacts with the hypochlorite, and chloramine is formed on the lysine side chain of the lacrimal proteins and amino of the lacrimal proteins; the chloramine is decomposed to form organic molecular fragments [1, 2] in a carbonyl form; and the peptide bonds on the lysine side chain are broken and decomposed into small molecular protein and amino acid, and thus the lacrimal proteins are degraded.

In a further embodiment, the hypochlorite reacts with the side chain of the peptide chain forming the lacrimal protein framework; the side chain further comprises one or more of a methionine side chain, a cysteine side chain, a histidine side chain, an $\alpha$-amino acid side chain, a tryptophan side chain, a tyrosine side chain, an arginine side chain and a glutamic acid side chain; and a reaction activity of the lysine side chain and the hypochlorite is between the reaction activity of the tryptophan side chain and the reaction activity of the tyrosine side chain.

In a further embodiment, a circuit power supply of the contact lens cleaner is switched on, then the two electrophoresis dissociation probes and the electrolyte solution form a conductive loop to generate current, and the two electrophoresis dissociation probes respectively form the anode and the cathode; and the charged lacrimal proteins in the electrolyte solution move to the electrode opposite to the electrical property thereof; positively charged hydrogen ions in the solution containing Cl⁻ move to the cathode and undergo a reduction reaction near the cathode so as to generate hydrogen; and negatively charged chloride ions in the solution containing Cl⁻ move to the anode in the solution and undergo an oxidation reaction to generate chlorine; chlorine reacts with water in the solution to generate hypochlorite; and the hypochlorite enables the lacrimal proteins to be degraded from the lens to be cleaned.

In a further embodiment, the solution containing Cl- comprises a solution containing Cl- with a concentration of 0.01-10%; and the solution containing Cl⁻ comprises one or more of a NaCl solution, a KCl solution, a $MgCl_2$ solution and a lens care solution containing Cl. It can be said that chlorate without metal ions harmful to human eyes can be used as the electrolyte solution of the invention.

In a further embodiment, the solution containing Cl- comprises a NaCl solution having a concentration of 0.9%.

On the other hand, the present disclosure further provides another method for protein removal and sterilization by electrophoresis dissociation. The method comprises: providing a contact lens cleaner comprising a lens cleaning tank having at least two electrophoresis dissociation probes arranged opposite each other therein; adding a lens to be cleaned and an electrolyte solution serving as a cleaning solution into the lens cleaning tank; forming two electrodes having opposite electrical properties by the two electrophoresis dissociation probes under a circuit loop, lacrimal proteins on the lens to be cleaned being charged in the electrolyte solution, and the charged lacrimal proteins moving towards the electrode opposite to the electrical property thereof; and enabling the electrolyte solution to react in the lens cleaning tank to generate a strong oxidant which undergoes an oxidation reaction with the lacrimal proteins, thereby degrading the lacrimal proteins.

According to the technical solution, the strong oxidant absorbs electrons of surface proteins on cell walls of microorganisms in the lens cleaning tank during decomposition, so that the surface proteins on the cell walls of the microorganisms are inactivated, and the microorganisms are killed; and the electrolyte solution comprises an ionic solution which contains chlorate and can be used as a lens care solution.

In another aspect, the present disclosure further provides a contact lens cleaner for implementing the method for protein removal and sterilization by electrophoresis dissociation. The contact lens cleaner comprises: a main machine shell, comprising a cleaning bin, a base, a lens cleaning tank being arranged in the cleaning bin, an integrated circuit being arranged in the base, and two electrophoresis dissociation probes in the lens cleaning tank being electrically connected to the integrated circuit. The cleaning bin is a magnetic control integrated bin. Each electrophoresis dissociation probe is an electrophoresis dissociation probe having a nano-scale coating material; the electrophoresis dissociation probe extends into the lens cleaning tank from a bottom of the lens cleaning tank, and the electrophoresis dissociation probe is close to an inner wall of the lens cleaning tank; and a top end of the electrophoresis dissociation probe is lower than an upper edge of a tank wall of the lens cleaning tank.

According to the technical solution, the integrated circuit comprises a power supply management circuit, a booster circuit and a switch; and the switch comprises a touch button arranged on the cleaning bin or the base; starting an electrophoresis dissociation protein removal function of the cleaner specifically comprises: manually touching the touch button to switch on the power supply management circuit to form a conductive loop and generate loop current, a voltage difference being formed by the loop current through the booster circuit; receiving the voltage difference by two electrophoresis dissociation probes in the cleaning tank; and forming an electrolytic tank in the lens cleaning tank through the electrolyte solution in the lens cleaning tank

Further, the power supply management circuit comprises a power supply; the power supply provides electric energy for the conductive loop; the two electrophoresis dissociation probes form an anode and a cathode in the lens cleaning tank after being electrified; the lacrimal proteins on the lens move towards a position of the electrode having the opposite electrical property in the electrolyte solution in the lens cleaning tank; and the electrolyte solution reacts in the lens cleaning tank to generate ions which react with the lacrimal proteins.

Compared with the prior art, the present disclosure has the following advantages and beneficial effects:

1. In the present disclosure, a protein electrophoresis principle and an electrolysis technology are combined to generate a novel electrophoresis dissociation protein removal and sterilization technology; with the technology and the electrolyte solution containing chloride ions, hypochlorite can be generated in the cleaning tank; the hypochlorite serving as the strong oxidant can absorb the electrons of the surface proteins on the cell walls of bacteria; and therefore, the functional proteins on the surfaces of the bacteria are oxidized, the bacteria will be inactivated finally due to incapability of absorbing nutrition, incapability of normal metabolism, and stopped splitting, so that the effects of sterilization and disinfection are achieved finally; the hypochlorite serving as the strong oxidant can further absorb the electrons of the surface proteins on the shells of the viruses, thereby inactivating the viruses; the hypochlorite can act on the surfaces of the microorganisms and can also permeate into the microorganism cells to undergo an oxidation reaction with bacteria (viruses) mycoprotein, nucleic acid, enzymes and other organic macromolecules in the microorganism cells, and thus pathogenic microorganisms are killed. Therefore, the method for protein removal and sterilization by electrophoresis dissociation of the present disclosure has dual effects of protein removal and sterilization.

2. In the present disclosure, the electrolyte solution used in the method for protein removal and sterilization by electrophoresis dissociation may be any solutions containing Cl⁻ and not having heavy metals, and even normal saline and common care solution may be used as the electrolyte solution of the present disclosure. Therefore, any available solution containing the chloride ions in the method for protein removal and sterilization by electrophoresis dissociation of the present disclosure can achieve the dual effects of protein removal and sterilization.

3. There will be deposited proteins on the contact lenses that have been used for a long time and in the cleaning tank. According to the method for protein removal and sterilization by electrophoresis dissociation of the present disclosure, with the lacrimal protein degrading property of hypochlorite, hypochlorite and the deposited proteins undergo an oxidation reaction to decompose the stubborn deposited proteins into small molecular proteins and amino acid; and the small molecular proteins are easier to adsorb than the deposited proteins. Therefore, the deposited proteins can be effectively removed by the method of the present disclosure.

4. According to the present disclosure, the lacrimal proteins can be degraded by hypochlorite, and the degradation of the lacrimal proteins by hypochlorite comprises the following two modes: 1. the framework peptide chain of the proteins is directly degraded; this degrading mode is to implement reaction between hypochlorite and the peptide framework to generate chloral formamide; and the framework in a form of chloral formamide is easily hydrolyzed in an aqueous solution, so that the peptide bonds will be broken, and the proteins will be degraded; and 2. the side chain on the peptide framework is degraded by hypochlorite; according to the arrangement based on the reaction activity of the side chain and hypochlorite, the side chain is methionine > cysteine > histidine > $\alpha$-amino acid > tryptophan > lysine > tyrosine > arginine > glutamic acid; unstable chloramine is generated on the amino and lysine side chains; and finally chloramine is decomposed to form the organic molecular fragments [1, 2] in the carbonyl form. According to the two reaction degradation modes, the lacrimal proteins are decomposed into the small molecular proteins to assist electrophoresis adsorption, so that the protein removal effect is more thorough. Of course, because of the diversity of protein molecules, there are more than two modes for degrading protein by hypochlorite. The present disclosure only provides the two relatively effective degradation modes.

5. When a user uses the method for protein removal and sterilization by electrophoresis dissociation of the present disclosure to clean the contact lens, the cleaning effect is related to the concentration of the chloride ions in the electrolyte solution, the cleaning duration, the voltage configured for the electrophoresis dissociation probes by the contact lens cleaner, the accumulated use time of the contact lens and the content of proteins deposited on the contact lens; the user can estimate the cleaning duration according to the use condition of the lens and the voltage configuration condition of the cleaner for cleaning; the cleaning can be finished within about 30 min under general normal conditions; of course, there is no adverse effect even if the cleaning time is long; and if the voltage of the cleaner is increased or the concentration of the chloride ions in the electrolyte solution is slightly increased, the cleaning effect will not be influenced even if the cleaning duration is shorter than 30 min.

6. In the present disclosure, the solution containing chloride ions is used in the method for protein removal and sterilization by electrophoresis dissociation; if the concentration of the chloride ions is high and the chloride ions are still in a supersaturated state after reaction, the chloride ions will generate a very small amount of chlorine, and the user generally covers the contact lens cleaner during cleaning; therefore, after the cleaning is completed, the user needs to open the cover of the cleaning tank and place the cleaner in a place with air circulation, and the cleaned contact lens can be used after a period of time; however, it is to be noted that the electrolyte solution in the present disclosure is the solution containing Cl⁻ with a concentration of 0.01-10% or normal saline, and the solution in this concentration range will not be in the supersaturated state after the reaction of the chloride ions generally; further, the electrolyte solution of the present disclosure may be the normal saline or the common care solution, and the effects are the same; therefore, under the same or almost the same effects, a situation of generating the harmful chlorine due to too high concentration of the CI- will not occur generally according to the purchase habits of consumers and the general lens cleaning and care matching habits.

7. The present disclosure discloses the method for protein removal and sterilization by electrophoresis dissociation; compared with a method for rubbing and soaking a lens with a care solution in the prior art, the protein removal method provided by the present disclosure prolongs the service life of the lens; under the support of the technical principle of electrophoresis dissociation protein removal and sterilization, the lens cleaning effect can be quantified through a laboratory detection result; the result shows that the present disclosure can remove protein nearly 100%; and such high-cleanliness cleaning method can reduce the risk of bacterial infection of human eyes.

8. According to the present disclosure, the lacrimal proteins on the lens are separated from the lens by the electrophoresis technology, and the electrolysis technology is utilized in match to degrade the lacrimal proteins (the protein is irreversibly degraded under an acidic or alkaline condition); the proteins are degraded to form a part which is easily dissolved in water or small molecules, so that the lacrimal proteins molecules are thoroughly removed; the hypochlorite in the electrolytic solution can also oxidize bacterial cell walls to achieve the sterilization and disinfection effects, serving multiple purposes; for a new contact lens or a lens with a short service cycle, the contact lens cleaner of the present disclosure can be used for sterilizing and disinfecting the new lens (because the lacrimal proteins on the new lens or the lens with a short service cycle is relatively less, the contact lens cleaner of the present disclosure mainly achieves the effects of sterilization and disinfection under this using condition); and for the lens with a long service cycle, more lacrimal proteins are inevitably deposited on the lens (because human eyes are a complex environment, the lens inevitably adsorbs more lacrimal proteins after long-term use). The contact lens cleaner of the present disclosure has dual effects of protein removal and sterilization when being used for cleaning the lens.

9. The method for protein removal and sterilization by electrophoresis dissociation of the present disclosure fundamentally solves the problem that the lacrimal proteins are difficult to remove since the advent of a corneal contact lens, greatly prolongs the service life of the corneal contact lens, reduces the cost of the user, and improves the safety coefficient of the user wearing the contact lens; the novel electrophoresis dissociation protein removal and sterilization technology is adopted and breaks a current situation that the contact lens industry depends on foreign countries from materials to technologies; and therefore, the protein removal and caring problem of the contact lens is thoroughly solved.

## BRIEF DESCRIPTION OF THE DRAWINGS

To describe the technical solutions in the embodiments of this present disclosure or the prior art more clearly, the accompanying drawings required for describing the embodiments or existing technologies are briefly described below. Apparently, the accompanying drawings in the following description show some embodiments of this present disclosure, and a person of ordinary skill in the art may still derive other drawings from these accompanying drawings without creative efforts.

FIG. 1 is a schematic diagram of a connection structure of a lens cleaning tank, an electrophoresis dissociation probe and a circuit power supply of a contact lens cleaner of the present disclosure in one embodiment (the electrophoresis dissociation probe drawn in the figure is long, and is higher than a top part of the cleaning tank, which is only used for highlighting the electrophoresis dissociation probe);

FIG. 2 is a schematic exploded view of an overall structure of a contact lens cleaner of the present disclosure in one embodiment;

FIG. 3 is an electrophoresis gel image obtained by detecting a degradation condition of a 3N care solution on lysozyme by 12% SDS-PAGE in a Verification Embodiment 2;

FIG. 4 is an electrophoresis gel image obtained by detecting a degradation condition of a commercial hard corneal contact lens protein removing solution on lysozyme by 15% SDS-PAGE in a Verification Embodiment 2;

FIG. 5 is a picture observed under an ultraviolet lamp after proteins are added to a lens and adsorbed at 25°C for 48 h in a Verification Embodiment 3; and a lens (a) on the left and a lens (b) on the right are experimental lenses under the same condition; and

FIG. 6 is a protein fluorescence detection diagram obtained after a contrast experiment carried out on a lens (a) and a lens (b) in FIG. 5; a lens (a1) on the left is obtained after electrophoresis carried out on the lens (a) in FIG. 5, and a lens (b1) on the right is obtained after electrophoresis carried out on the lens (b1) in FIG. 5.

In the drawings: 1-cleaning bin; 2-base; 10-lens cleaning tank; 20-electrophoresis dissociation probe; and 30-integrated circuit.

## DETAILED DESCRIPTION OF THE INVENTION

The following clearly and completely describes the technical solutions in the embodiments of the present disclosure with reference to the accompanying drawings in the embodiments of the present disclosure. Apparently, the described embodiments are some of the embodiments of the present disclosure rather than all embodiments. All other embodiments obtained by a person of ordinary skill in the art based on the disclosed embodiments without creative efforts shall fall within the protection scope of the present disclosure.

In the description of the present disclosure, it should be understood that orientation or position relationships indicated by the terms such as "upper", "lower", "top", "bottom", "inner" and "outer" are based on orientation or position relationships shown in the accompanying drawings, and are used only for ease and brevity of illustration and description, rather than indicating or implying that the mentioned apparatus or component need to have a particular orientation or need to be constructed and operated in a particular orientation. Therefore, such terms should not be construed as limiting of the present disclosure. In the descriptions of the present disclosure, unless otherwise explicitly specified, "multiple" means two or more than two.

In the present disclosure, it should be noted that unless otherwise explicitly specified and limited, the terms "mount", "connect", "connection", and "fix" should be understood in a broad sense. For example, a connection may be a fixed connection, a detachable connection, or an integral connection; or the connection may be a mechanical connection or an electrical connection; or the connection may be a direct connection, an indirect connection through an intermediate medium, internal communication between two components, or an interaction relationship between two components. A person of ordinary skill in the art may understand the specific meanings of the foregoing terms in the present disclosure according to specific situations.

The present disclosure will be further illustrated as follows in conjunction with the accompanying drawings and the embodiments.

### Embodiments

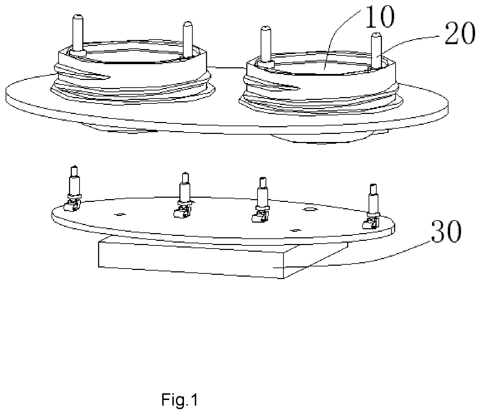

As shown in FIG. 1, it shows a partial schematic structure of a contact lens cleaner having an electrophoresis dissociation protein removal function. The contact lens cleaner comprises a plurality of lens cleaning tanks 10. Two electrophoresis dissociation probes 20 are arranged in each lens cleaning tank 10 opposite each other. The electrophoresis dissociation probe 20 is a nano-scale coating material electrophoresis dissociation probe. The electrophoresis dissociation probe 20 extends into the lens cleaning tank 10 from a bottom of the lens cleaning tank 10, and the electrophoresis dissociation probe 20 is close to an inner wall of the lens cleaning tank 10. A top end of the electrophoresis dissociation probe 20 is lower than an upper edge of a tank wall of the lens cleaning tank 10.

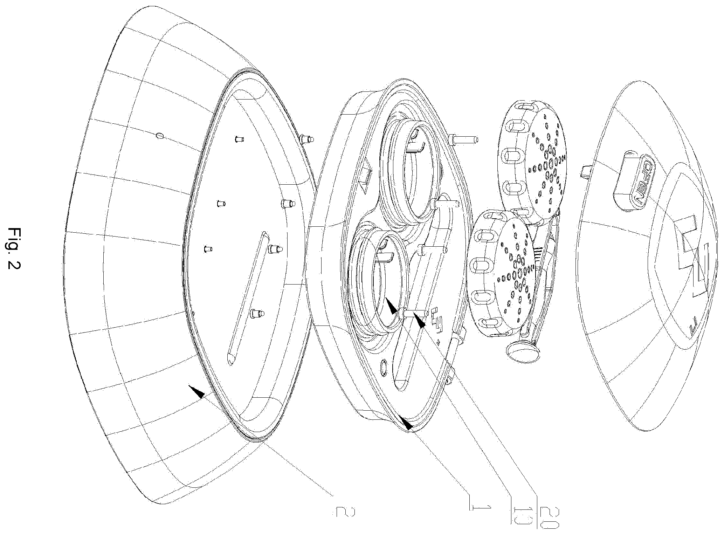

In one embodiment, as shown in FIG. 2, the contact lens cleaner of the present disclosure further comprises a main machine shell. The main machine shell comprises a cleaning bin 1 and a base 2. The lens cleaning tank 10 is arranged in the cleaning bin 1. An integrated circuit 30 is arranged in the base 2. The electrophoresis dissociation probe 20 in the lens cleaning tank 10 is electrically connected to the integrated circuit 30.

In one embodiment, the cleaning bin 1 of the present disclosure is a magnetic control integrated bin.

In one embodiment, the integrated circuit 30 of the present disclosure comprises a power supply management circuit, a booster circuit and a switch. The switch comprises a touch button arranged on the cleaning bin 1 or the base 2.

The power supply management circuit comprises a power supply. The power supply provides electric energy for a conductive loop. The two electrophoresis dissociation probes form an anode and a cathode in the lens cleaning tanks after being electrified. Charged lacrimal proteins on a lens move towards a position of the electrode opposite to the electrical property thereof in an electrolyte solution in the lens cleaning tank so as to form an electrophoresis phenomenon. The electrolyte solution reacts in the lens cleaning tank 10 (or in the whole lens cleaning tank 10, particularly near the electrode), thereby obtaining ions capable of reacting with the lacrimal proteins.

Starting an electrophoresis dissociation protein removal function of the cleaner specifically comprises: manually touching the touch button to switch on the power supply management circuit to form a conductive loop and generate a loop current, a voltage difference being formed by the loop current through the booster circuit; receiving the voltage difference by two electrophoresis dissociation probes in the cleaning tank; and forming an electrolytic tank in the cleaning tank through the electrolyte solution in the cleaning tank. Under an experiment condition, the time for completely electrolyzing and dissociating the lacrimal proteins on a lens to be cleaned through the electrolytic tank is 30 min. The time can be obtained through later verification embodiments; and it can be detected that the protein is completely removed after 30 min.

In one embodiment, the present disclosure provides a method for cleaning a lens by the contact lens cleaner. The method comprises the following steps:

opening a cover arranged on the lens cleaning tank 10 in the contact lens cleaner;

filling the lens cleaning tank 10 with the electrolyte solution, in one embodiment, the electrolyte solution being a neutral solution, such as a 0.9% NaCl solution (namely normal saline which does not have disinfection and sterilization functions); putting a lens to be cleaned into the lens cleaning tank 10;

closing the cover of the lens cleaning tank 10;

selecting cleaning voltage and cleaning duration, starting the switch (the touch button) of the contact lens cleaner to enable the contact lens cleaner to start protein removal and sterilization; and

opening the cover of the lens cleaning tank 10 after cleaning is completed. The lens can be used after being flushed or directly worn.

It is to be noted that the solution containing chloride ions is used in the method for protein removal and sterilization by electrophoresis dissociation of the present disclosure. If the concentration of the chloride ions is high and the chloride ions are still in a supersaturated state after reaction, the chloride ions will generate a very small amount of chlorine, and the user generally covers the contact lens cleaner during using. Therefore, after the cleaning is completed, the user needs to open the cover of the cleaning tank and place the cleaner in a place with air circulation, and the cleaned contact lens can be used after 10-15 min. It is to be noted that the electrolyte solution in the present disclosure is a solution containing Cl- with a concentration of 0.01-10% or normal saline, and the solution in this concentration range will not be in the supersaturated state after the reaction of the chloride ions generally. Furthermore, the electrolyte solution of the present disclosure may be the normal saline or the common care solution, and the effects are the same. Therefore, under the same or almost the same effects, a situation of generating harmful chlorine due to too high concentration of the Cl- will not occur generally according to the purchase habits of consumers and the general lens cleaning and care matching habits. Finally, according to the actual using condition of the user, this situation will not occur generally because the contact lens has a using period, such as a lens with service life of a month and a lens with service life of three months, which are not very dirty and need strong protein removal. Therefore, the consumer will not use the solution which is not in a reasonable concentration range for cleaning and caring.

However, in order to guarantee the life health safety of the user to the maximum extent, it is to be noted herein that: if the user uses a slightly alkaline electrolyte solution containing chloride ions, it is better to open the cover at the place with air circulation and stand for 10-15 min, and then flush the contact lens with clear water, thereby preventing a situation that the concentration of the chloride ions is too high, a small amount of chlorine is generated, and consequently the human eyes are possibly stung.

Further, when the user uses the method for protein removal and sterilization by electrophoresis dissociation of the present disclosure to clean the contact lens, the cleaning effect is related to the concentration of the chloride ions in the electrolyte solution, the cleaning duration, the voltage configured for the electrophoresis dissociation probes by the contact lens cleaner, the accumulated use time of the contact lens and the content of proteins deposited on the contact lens. The user may also select the cleaning duration freely according to the use condition of the lens and the voltage configuration condition of the cleaner for cleaning. The cleaning can be finished within about 30 min under general normal conditions. Of course, there is no adverse effect even if the cleaning time is long or short. If the voltage of the cleaner is increased or the concentration of the chloride ions in the electrolyte solution is slightly increased, the cleaning effect will not be influenced even if the cleaning duration is shorter than 30 min (moreover, human eyes will secrete some substances capable of sterilizing; and under the normal condition, if the cleaning time is not long enough, a small quantity of microorganisms remaining on the lenses will be killed by the sterilizing substances secreted by the human eyes, so that it will not be a big problem if the cleaning time is too short).

For the cleaning effect, it is to be emphasized that the method for protein removal and sterilization by electrophoresis dissociation of the present disclosure is a superposition technology of electrophoresis and electrolysis. The cleaning effect is also the protein removal and sterilization effect realized by the electrophoresis and the electrolysis together. Therefore, the lens cleaning effect is related to the concentration of Cl- contained in the electrolyte solution, the electrolysis voltage, the electrophoresis electrolysis duration and the quantity of the lacrimal proteins attached to the lens. For example, for cleaning the lens, a solution containing high-concentration Cl⁻ is used as the electrolyte solution, then high voltage is utilized for short-time electrophoresis electrolysis, so that the solution containing high-concentration Cl⁻ will not generate hypochlorite having overhigh concentration in the solution, and further, the effective cleaning can be carried out within shortened cleaning time. It is particularly suitable for a user in a hurry. In addition, even if the hypochlorite having overhigh concentration is generated in the solution, the user can open the cover at the place with air circulation after the cleaning is completed, and the lens can be directly flushed with running water for less than 1 min and be directly worn. Similarly, if the consumer uses a solution containing extremely-low-concentration Cl- to clean the lenses, the cleaning can also be carried out for a long time under relatively low voltage, for example, 40-50 min. It is a cleaning solution more suitable for the user with abundant time. Generally, the lens cleaned with the solution containing extremely-low-concentration Cl⁻ can be almost directly worn after the cleaning is completed.

The principle of electrophoresis dissociation protein removal in the present disclosure is that: the method for protein removal and sterilization by electrophoresis dissociation of the present disclosure is mainly to superpose the protein electrophoresis technology and the electrolysis technology. First, the protein electrophoresis technology refers to a phenomenon that charged particles or molecules move in an electric field. Macromolecular protein, polypeptide, virus particles, even cells or micromolecular amino acid and nucleoside and the like can perform directional electrophoresis in the electric field. Under the action of the contact lens cleaner of the present disclosure, the lacrimal proteins are charged and move towards the positions of the electrodes of the probe under the action of the field intensity of the probe in the lens cleaning tank of the cleaning bin, so that the effect of cleaning the corneal contact lens is achieved. Moreover, the electrolysis technology refers to a process that after current passes through the electrolyte solution or a molten electrolyte (also known as an electrolyte), a redox reaction is caused on the cathode and the anode.

In one embodiment, a Nacl solution is used as the electrolyte solution for cleaning the lens in the prevent disclosure, the chloride ions in the Nacl solution move to the anode probe in the electrolysis process, lose electrons and are reduced into chlorine. One part of chlorine generated by the Nacl solution having a certain concentration will be discharged in a form of bubbles, and the other part of the chlorine is dissolved in water and undergoes the following chemical reaction:

$$Cl2+H2O=HCl+HClO$$

wherein HClO generated in this reaction has the effects of degrading protein, sterilizing and disinfecting.

The theory above proves that the electrolyte solution for implementing the method for protein removal and sterilization by electrophoresis dissociation in the present disclosure may be any chloride ion containing solution which contains no heavy metals, and even normal saline and common care solution (basically all the care solutions in the market contain the chloride ions) can be used as the electrolyte solution in the present disclosure. The more conventional ones, such as a NaCl solution, a KCl solution, an $MgCl_2$ solution and a lens care solution containing Cl⁻ or a combination of the above-mentioned solutions (on the premise that there is no reaction which will damage electrolysis, and consequently, destroy the generation of hypochlorite in the lens cleaning tank), or, overall, an ionic solution which contains chlorate and can be used as the lens care solution can be used as the electrolyte solution in the present disclosure. Therefore, any available solution containing the chloride ions in the method for protein removal and sterilization by electrophoresis dissociation of the present disclose can achieve dual effects of protein removal and sterilization.

In the present disclosure, the two electrophoresis dissociation probes 20 in the lens cleaning tank 10 are connected to the power supply through the electrolyte solution to form a conductive loop, current is generated, and the two electrophoresis dissociation probes 20 form two electrodes (one anode and one cathode) having different electrical properties.

There are lacrimal proteins on the surface of the lens to be cleaned, and the lacrimal proteins are charged in the electrolyte solution (generally, if the electrolyte solution is a solution having the PH value higher than an isoelectric point of the lacrimal proteins, the lacrimal proteins will be charged in the electrolyte solution due to negative charges; and if the electrolyte solution is a solution having the PH value lower than an isoelectric point of the lacrimal proteins, the lacrimal proteins will be charged in the electrolyte solution due to positive charges). The charged lacrimal proteins move to the electrode opposite to the electrical property thereof in electrical property in the cleaning tank according to the electrophoresis phenomenon.

In one embodiment, when the method for protein removal and sterilization by electrophoresis dissociation of the present disclosure is used for cleaning the lens, the used electrolyte solution is neutral and slightly alkaline.

In the present disclosure, the electrophoresis dissociation probe 20 of the contact lens cleaner is an electrophoresis dissociation probe having a nano-scale coating material. The material of the electrophoresis dissociation probe is corrosion-resistant, so that the redox reaction caused by electrolysis in the cleaning tank will not cause electrode corrosion; or, the electrolyte solution does not react with the electrophoresis dissociation probe, the electrolyte solution reacts near the two electrodes having different electrical properties (from the perspective of concentration, according to theory of directional movement of the ions, and compared with other positions in the lens cleaning tank 10, the concentration of ions near the electrodes is high; however, the positions in the lens cleaning tank 10 is originally small, and the concentration of the electrolyte is fixed, i.e.,, the reaction is performed in the lens cleaning tank 10), and ions damaging the molecular structure of lacrimal proteins are formed, so that the lacrimal proteins are electrolyzed and dissociated by the ions.

The technical principle of protein removal by electrophoresis dissociation is specifically illustrated as follows in conjunction with the contact lens cleaner.

The electrophoresis dissociation probe 20 is arranged at a position close to an inner wall of the lens cleaning tank 10. The lens to be cleaned is placed in the lens cleaning tank 10. If the circuit power supply is switched on, the two electrophoresis dissociation probes 20 in the lens cleaning tank 10 and the electrolyte solution form the conductive loop, and thus the lacrimal proteins on the lens to be cleaned generate an electrophoresis phenomenon in the electrolyte solution.

The two electrophoresis dissociation probes 20 in the lens cleaning tank 10 and the electrolyte solution form the conductive loop. The conductive loop generates current. The electrolyte solution and the two electrodes having different electrical properties undergo a redox reaction under the action of the current so as to generate ions having strong oxidizing properties (according to the embodiment of the present disclosure, it can be understood as referring to hypochlorite generated in the solution). The ions and peptide bonds forming the lacrimal proteins undergo an oxidation reaction, and thus the lacrimal proteins molecules are degraded.

The two electrophoresis dissociation probes 20 in the lens cleaning tank 10 and the electrolyte solution form the conductive loop. The conductive loop generates current. Under the action of the current, one of the two electrophoresis dissociation probes 20 forms the anode (anode electrode), and the other forms the cathode (cathode electrode).

The lacrimal proteins are charged in the electrolyte solution. The charged lacrimal proteins move to the electrode opposite to the electrical property thereof in the cleaning tank. In the moving process, the lacrimal proteins fall off from the surface of the lens to be cleaned and gather at the electrode opposite to the electrical property thereof in the lens cleaning tank 10, so that the electrophoresis phenomenon is realized.

In one embodiment, the electrolyte solution comprises a solution containing NaCl. The solution containing NaCl is electrolyzed under the action of current. Sodium ions and hydrogen ions in the solution move to the cathode electrode. The chloride ions move to the anode electrode.

The hydrogen ions gaining the electrons from the cathode electrode undergo a reduction reaction to generate hydrogen. The chloride ions release electrons at the anode electrode and undergo an oxidation reaction to generate chlorine. The chlorine reacts with water in the solution to generate hypochlorite.

Cl- in the electrolyte moves to the anode of the probe in the Nacl electrolysis process, loses the electrons and is reduced into $Cl_2$. One part of the generated chlorine is discharged in the form of bubbles, and the other part of the generated chlorine is dissolved in water and undergoes chemical reaction: Cl2+H2O=HCl+HClO.

HClO has the effect of degrading protein and can decompose deposited proteins on the corneal contact lens into small molecular proteins and amino acid, so that the removal of the lacrimal proteins on the corneal contact lens is facilitated. The degradation reaction mainly has two mode: 1. The framework peptide chain of the proteins is directly degraded; in this mode, the hypochlorite reacts with the peptide framework to generate chloral formamide; and the framework in a form of chloral formamide is easily hydrolyzed in an aqueous solution, so that the peptide bonds are broken, and the proteins are degraded; and 2. The side chain on the peptide framework is degraded, and the reaction activity is methionine > cysteine > histidine > $\alpha$-amino acid > tryptophan > lysine > tyrosine > arginine > glutamic acid; unstable chloramine is generated on the amino and lysine side chains; and finally chloramine is decomposed to form organic molecular fragments [1, 2] in the carbonyl form.

According to the above protein removal principle, predictably, stubborn deposited proteins on the contact lens with long service cycle and in the cleaning tank can undergo an oxidation reaction with the hypochlorite so as to be decomposed into small molecular proteins and amino acid by the dissociation protein removal method of the present disclosure. The small molecular proteins are easier to adsorb than the deposited proteins. Therefore, the deposited proteins can be effectively removed by the method of the present disclosure.

Meanwhile, HClO also has the effects of sterilization and disinfection: The hypochlorite will slowly undergo self-redox reaction so as to be decomposed. Each hypochlorite molecule will absorb the electrons during decomposition (the hypochlorite serving as a strong oxidant can absorb the electrons of surface proteins on microbial cell walls so as to oxidize the functional proteins on the microorganism surfaces, and the microorganisms will be inactivated due to incapability of absorbing nutrition, incapability of normal metabolism, and stopped splitting finally, so that the effects of sterilization and disinfection are achieved finally), and thus the aim of sterilization is fulfilled. Hydrogen ions, chloride ions and oxygen are dissociated. The chemical formula is 2HClO → 2H+ + 2Cl- + O2↑.

The hypochlorite can act on the cell walls and the virus shells in the sterilizing and virus killing process. Moreover, because the hypochlorite is micromolecular and free of charges, the hypochlorite can permeate into bacteria (viruses) and undergo an oxidation reaction with bacteria (viruses) mycoprotein, nucleic acid, enzymes and other organic macromolecules to kill pathogenic microorganisms.

Compared with a method for rubbing and soaking a lens with a care solution in the prior art, the method for protein removal and sterilization by electrophoresis dissociation disclosed by the present disclosure has the advantages that the service life of the lens is prolonged, the proteins can be effectively removed, and sterilization and disinfection can be realized; and a risk that human eyes are infected by bacteria can be reduced by the high-cleanliness cleaning method with dual protection.

For a new contact lens or a lens with a short service cycle, less or almost no lacrimal proteins are attached to the lens, so that the contact lens cleaner of the present disclosure can be used for sterilizing and disinfecting new lens. For the lens with long service cycle, more lacrimal proteins are inevitably deposited on the lens (because human eyes are a complex environment, the lens inevitably adsorbs more lacrimal proteins after long-term use). The contact lens cleaner of the present disclosure has dual effects of protein removal and sterilization when being used for cleaning the lens.

**Verification Embodiment 1:**

The cleaning effect of the contact lens cleaner on protein was verified.

A removing experiment was performed for lysozyme by the electrophoresis dissociation protein removal function of the contact lens cleaner of the present disclosure (the following experimental contents and data were tested and provided by a BIOTOP Biomedical Public Service Platform of SIP BioBay).

A protein solution having a certain concentration was prepared; a 0.9% NaCl solution was added into the contact lens cleaner of the present disclosure for an electrophoresis experiment. Hypochlorite was generated in the process, which could degrade the proteins. It is to be noted that in main equipment used in the following description, a 3N corneal contact lens electrophoresis dissociation protein removal and sterilization instrument is the contact lens cleaner of the present disclosure. In order to facilitate description, the contact lens cleaner is called as the 3N corneal contact lens electrophoresis dissociation protein removal and sterilization instrument in the following.

Main equipment: 1. 3N corneal contact lens electrophoresis dissociation protein removal and sterilization instrument. 2. Micro UV spectrophotometer: Nanodrop One/Onec.

Main reagent solution: 1. Protein: lysozyme, Solarbio (Article No.: L8120), molecular weight: 14 KDa; 2. 0.9% NaCl was used for preparing; the concentration was tested; 2 ml was taken for electrophoresis for 30 min; and then the concentration after electrophoresis was detected.

The hypochlorite generated in electrophoresis will influence ultraviolet absorption of A280, and 10% sodium sulfite can be added to eliminate the influence thoroughly. In order to enable detected proteins to be under the same background, when the proteins were subjected to electrophoresis, a 0.9% NaCl solution without proteins was set as a parallel control and subjected to electrophoresis for 30 min; 10% sodium sulfite was added; and the solution was used for background deduction.

Experimental results: the protein concentrations were tested 3 times, and the average concentration was used as the final concentration.

The concentration in the first 3 times of electrophoresis was respectively: 657.4 $\mu$g/mL; 651.2 $\mu$g/mL; 649.8 $\mu$g/mL; the average concentration was 652.8 $\mu$g/mL; 1 mL of the solutions was taken after 30 min of electrophoresis; 1 mL of 20% sodium sulfite was added, and the average concentration of the contact lens cleaner was 99.9 $\mu$g/mL, 96.1 $\mu$g/mL, and 98.9 $\mu$g/mL; the average concentration was 98.3 $\mu$g/mL; and because this concentration was the concentration of sodium sulfite after being diluted by double, the final concentration was multiplied by 2 and was 196.6 $\mu$g/mL.

Protein degradation rate: (original concentration - residual concentration)/original

concentration x 100%=(652.8 µg/mL-196.6 µg/mL)/ 652.8 µg/mL=70%.

After 30 min of electrophoresis dissociation, 15 $\mu$L was taken from a cleaning bin for sample loading, and the protein sample loading amount was 9 $\mu$g; lysozyme was not detected by SDS-PAGE (biological electrophoresis gel running), and the proteins were completely decomposed, namely, the protein removal rate could reach 100% after 30 min of electrophoresis.

Therefore, it is found from the above experimental data that the method for protein removal by electrophoresis dissociation of the present disclosure can comprehensively remove the proteins, and the removing rate can reach 100%.

**Verification Embodiment 2:**

The protein degradation effects of different care solutions were verified.

**1. Protein degradation by 3N care solution:**

A 3N care solution could generate hypochlorite in normal saline under an electrophoresis condition so as to degrade the proteins. In order to make the detection result visual, theoretical concentration of lysozyme in artificial tear was improved from 1.9 mg/mL to 3 mg/mL.

Main equipment: 3N corneal contact lens electrophoresis dissociation protein removal and sterilization instrument.

Electrophoresis equipment: Bio-Rad Mini.

Main reagents:

(1). Protein: lysozyme, Solarbio (Article No.: L8120), molecular weight: 14 KDa; 0.9% NaCl was used for preparing, 3 mg/mL; and 3 μL was taken for sample loading, and the protein sample loading amount was 9 μg;

(2). Normal saline prepared by BIOTOP; lysozyme was prepared to reach 0.6 mg/mL, and 2 ml was added for electrophoresis for 30 min; and then 15 μL was taken for sample loading; and the protein sample loading amount was 9 μg;

(3). 3N special cleaning solution (0.9% NaCl solution); lysozyme was prepared to reach 0.6 mg/mL, and 2 ml was added for electrophoresis for 30 min; and then 15 μL was taken for sample loading; and the protein sample loading amount was 9 μg;

(4). Protein molecular weight standard: Thermo Fisher, Article No.: 26619.

(5). SDS-PAGE electrophoresis buffer: Shanghai Sangon Biotech, Article No.: C520001-0500.

(6). SDS-PAGE protein electrophoresis sample loading buffer: Shanghai Sangon Biotech: Article No.: C508320-0001.

(7). SDS-PAGE staining solution: Shanghai Sangon Biotech, Article No.: C900300-0100.

(8). SDS-PAGE bleaching solution: Shanghai Sangon Biotech, Article No.: C900299-0300.

(9). SDS-PAGE gel: Shanghai Sangon Biotech, Article No.: C661102-0001.

① Lysozyme: 0.9% NaCl was used for preparing, 3 mg/mL; and 3 μL was taken for sample loading, and the protein sample loading amount was 9 μg;

(2) Lysozyme: 0.9% NaCl was used for preparing, 3 mg/mL; normal saline was use for soaking for 30 min for gel running; and the protein sample loading amount was 9 μg;

③ 0.6 mg/mL lysozyme was prepared with normal saline, and 2 ml was added for electrophoresis for 30 min, and then 15 μL was taken for sample loading; and the protein sample loading amount was 9 μg;

④ Lysozyme was prepared to reach 0.6 mg/mL through the 3N special cleaning solution (0.9% NaCl solution), 2 ml was added for electrophoresis for 30 min, and then 15 μL was taken for sample loading. The protein sample loading amount was 9 μg; and

⑤ Protein molecular weight standard.

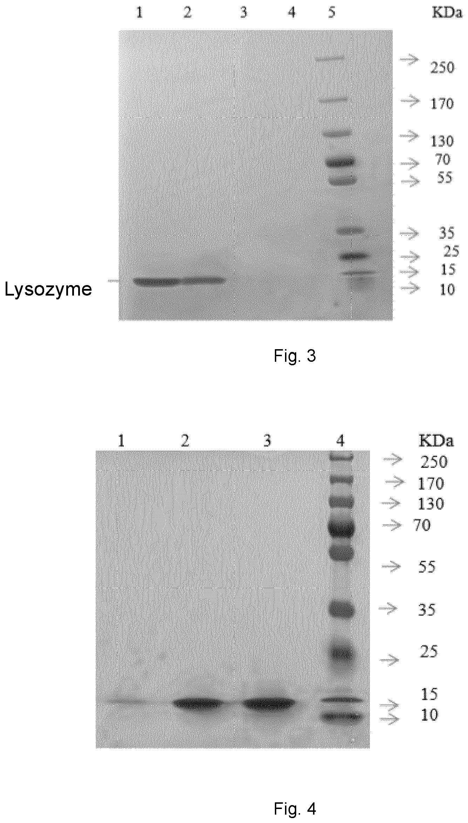

**Experimental results:** FIG. 3 is an electrophoresis gel image obtained by detecting a degradation condition of a 3N care solution on lysozyme by 12% SDS-PAGE; and it can be seen from the figure that the proteins are degraded by lysozyme prepared through 0.9% NaCl and the 3N special cleaning solution (0.9% NaCl solution), and no protein band is found.

**2. Protein degradation by other commercially available care solutions:**

Main reagents:

Care solution A: a combination solution for removing protein within 30 min of some brand;

Care solution B: some hydrogen peroxide for removing protein within 2 h.

(1). Protein: lysozyme, Solarbio (Article No.: L8120), molecular weight: 14 KDa;

(2). Sample treatment of care solution A: lysozyme was prepared to reach 0.6 mg/mL, 2 ml was added for soaking for 30 min, and then 15 μL was taken for sample loading; and the protein sample loading amount was 9 μg; Sample treatment of care solution B: lysozyme was prepared to reach 0.6 mg/mL, 2 ml was added for soaking for 2 h, and then 15 μL was taken for sample loading; and the protein sample loading amount was 9 μg;

(3). Protein molecular weight standard: Thermo Fisher, Article No.: 26619.

(4). SDS-PAGE electrophoresis buffer: Shanghai Sangon Biotech, Article No.: C520001-0500.

(5). SDS-PAGE protein electrophoresis sample loading buffer: Shanghai Sangon Biotech: Article No.: C508320-0001.

(6). SDS-PAGE staining solution: Shanghai Sangon Biotech, Article No.: C900300-0100.

(7). SDS-PAGE bleaching solution: Shanghai Sangon Biotech, Article No.: C900299-0300.

(8). SDS-PAGE gel: Shanghai Sangon Biotech, Article No.: C661103-0001.

① Care solution A: lysozyme was prepared to reach 0.6 mg/mL, 2 ml was added for soaking for 30 min, and then 15 μL was taken for sample loading; and the protein sample loading amount was 9 μg;

② Care solution B: lysozyme was prepared to reach 0.6 mg/mL, 2 ml was added for soaking for 2 h, and then 15 μL was taken for sample loading; and the protein sample loading amount was 9 μg;

③ 0.6 mg/mL lysozyme in the care solution B was prepared, 15 μL was taken for sample loading immediately; and the protein sample loading amount was 9 μg;

④ Protein molecular weight standard.

**Experimental results:** FIG. 4 is an electrophoresis gel image obtained by detecting a degradation condition of a commercially available hard corneal contact lens protein removing solution on lysozyme by 15% SDS-PAGE. It can be seen that with lysozyme in the care solution A prepared to reach 0.6 mg/mL, most of the proteins is degraded after soaking treatment is carried out for 30 min, and only weak strips remain; and with soaking treatment in the care solution B for 2 h, the protein strips are hardly degraded compared with a control group.

In conclusion, by comparing the degradation conditions of the 3N care solution and other commercially available hard corneal contact lens protein removing solutions on lysozyme, it can be found that the care solution or the electrolyte solution for removing protein is key to the final protein degrading condition; and the protein removing effect of the 3N care solution is better than that of other care solutions.

**Verification Embodiment 3:**

The protein removing effect of the contact lens cleaner of the present disclosure on the lens was verified.

In order to conveniently observe the residue condition of the proteins on the lens, green fluorescent proteins were used to detect the electrophoresis removing effect of the 3N corneal contact lens electrophoresis dissociation protein removal and sterilization instrument.

Main equipment: 3N corneal contact lens electrophoresis dissociation protein removal and sterilization instrument.

Contact lens: Eyebright, Article No.: DS200114030 /DS200114028.

Main reagents: Green fluorescent protein (GFP, GenBank: AAA27721.1), which is derived from Aequorea victoria, recombined and expressed in Escherichia coli and has a total length of 238 amino acids and a molecular weight of 27 kDa.

The electrophoresis solution is SDS-PAGE 10X Tris-Glycine electrophoresis buffer of Shanghai Sangon Biotech; Article No.: C520001-0500.

Sample treatment: 20 μL of 0.5 mg/mL GFP was taken and put on the lens, namely, the content of proteins on the lenses was 10 μg. The lens was placed a dark place at 25°C for 48 h, so that the proteins could be fully adsorbed onto the lens. Then electrophoresis was carried out by the 3N corneal contact lens electrophoresis dissociation protein removal and sterilization instrument for 10 min.

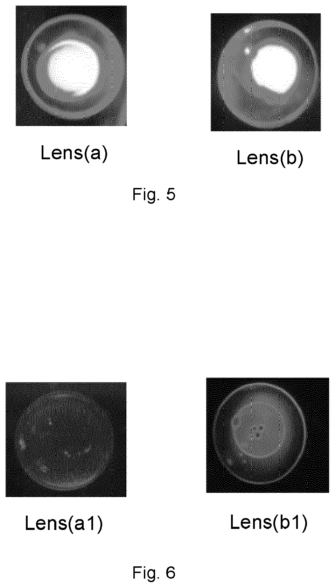

Two groups of lenses with the proteins were provided, namely the proteins were added onto the lenses; after absorption was carried out at 25°C for 48 h, strong green fluorescence could be observed on the lenses under an ultraviolet lamp. As shown in FIG. 5, the lenses on the left and right are the same and are used for contrast experiment later.

The lens on the left in FIG. 5 was subjected to electrophoresis through the 3N corneal contact lens electrophoresis dissociation protein removal and sterilization instrument in cooperation with the 3N care solution (electrophoresis solution); the lens on the right in FIG. 5 was soaked in the 3N care solution; the fluorescence conditions were compared under the ultraviolet lamp to obtain FIG. 6. The lens in FIG. 6 corresponds to the lens in FIG. 5. After the lens on the left was subjected to electrophoresis for 10 min, green fluorescence on GFP proteins disappeared; and after the lens on the right was soaked for 10 min, strong fluorescence still existed. Therefore, it is indicated that the 3N corneal contact lens electrophoresis dissociation protein removal and sterilization instrument can remove the green fluorescent proteins on the lenses through electrophoresis under this system.

**Verification Embodiment 4:**

The bacteria killing effect of the contact lens cleaner of the present disclosure was verified.

The electrophoresis dissociation protein removal function of the contact lens cleaner of the present disclosure was performed in cooperation with the 0.9% NaCl solution, and liquid subjected to electrophoresis dissociation for 3 min was detected for sterilization; the sterilization rate of the liquid for Escherichia Coli, Staphylococcus Aureus and Pseudomonas Aeruginosa reached 99.999%; and the sterilization rate of the liquid for Candida Albicans reached 99.99%.

It is indicated that the electrophoresis dissociation technology of the contact lens cleaner of the present disclosure used in cooperation with the electrolyte solution can achieve the sterilization and disinfection effects, and further, the risk that human eyes are infected with bacteria is avoided.

**Verification Embodiment 5:**

The bacteria killing effect of the contact lens cleaner of the present disclosure was verified.

Cysts containing Acanthamoeba castellanii (ATCC 30868 Acanthamoeba castellanii) and trophozoites PBS were respectively treated by the corneal contact lens protein electrophoresis dissociation removal and sterilization instrument (contact lens cleaner), a disinfectant having standard working concentration, and common normal saline; the mixed solution was collected; the survival rate of polypide in the collected mixed solution was judged by virtue of morphology observation such as an iodine staining method and an iron hematoxylin staining method, a mouse subjected to intraperitoneal inoculation was cultured by an ATCC medium 712; the survival rates of the Acanthamoeba castellanii and the cysts after being treated by the corneal contact lens electrophoresis dissociation protein removal and sterilization instrument, the disinfectant having the standard working concentration and the common normal saline were respectively calculated and were then compared with the morphological observation result for verification; and finally, the test was repeated 3 times to evaluate the killing effects of the corneal contact lens electrophoresis dissociation protein removal and sterilization instrument and the standard disinfectant (it has corrosivity and cannot used for disinfection of conventional contact corneal tools) and the normal saline (serving as negative control) on the Acanthamoeba castellanii.

Preferably, molecular biological identification was carried out on the ATCC standard strain of Acanthamoeba castellanii so as to verify the strain of the Acanthamoeba castellanii protozoa.

**Experiment steps:**

Preparation of articles: 5 ml disposable empty needles; sterile bacterial culture bottles; 15 ml centrifuge tube; ATCC culture mediums 712; iron hematoxylin iron related staining solutions; and glass slides.

Resuscitation:

(1) A sterile operating table was disinfected by ultraviolet for 20 min, and the temperature of a water bath tank was adjusted to be 28°C;

(2) Acanthamoeba castellanii cryopreservation tube was taken out and rapidly unfroze in the water bath tank;

(3) The molten Acanthamoeba castellanii suspension was sucked by an empty needle and added into a centrifuge tube; 2 ml of ATCC culture mediums 712 was sucked by another empty needle and added into the cryopreservation tube; and the residual Acanthamoeba castellanii was blown, beat and cleaned, and then transferred into the centrifuge tube;

(4) centrifuging was carried out at 500 rpm for 7 min;

(5) 4 ml of the culture mediums was added into the centrifuge tube; cells were blown and beat to generate a suspension; the suspension was uniformly divided and placed into two culture bottles; the culture medium was added into each bottle until reaching 5 ml; and then bottle caps were screwed;

(6) the two culture bottles were gently shaken at 28°C for 5 min so as to uniformly disperse the gathered cysts;

(7) horizontal incubation culture was carried out at 28°C (the culture bottles could be wrapped with sterile gloves and sealed with adhesive tapes to prevent pollution); and

(8) the suspension was replaced within 3-5 d, and subculture was carried out after the trophozoites were full.

**Liquid replacement or subculture:**

(1) Suction tubes or the empty needles were used for slightly blowing and beating to make a small amount of trophozoites suspend (the step could be omitted if replacing liquid only);

(2) The suspension was transferred into the centrifuge tubes, and centrifuged at 500 rpm for 7 min; and

(3) New culture mediums were added and blown and beat into a suspension, and the suspension was transferred into the culture bottle.

**In-vitro observation of influence of different treatments on Acanthamoeba castellanii:**

(1) Three different treatments and iron hematoxylin staining on Acanthamoeba castellanii:
Acanthamoeba castellanii in the culture bottle was respectively treated by the corneal contact lens electrophoresis dissociation protein removal and sterilization instrument (Experimental Group), the disinfectant having (Control Group) and common normal saline (Negative Control), and the survival rate of the Acanthamoeba castellanii was observed. The treated Acanthamoeba castellanii was subjected to iron hematoxylin staining by a normal saline smear method, and the activity change of Acanthamoeba castellanii trophozoite under the visual field was observed.

(2) Iron hematoxylin staining method:

① 0.5% hematoxylin solution, 4% and 2% iron alum solution and a Schaudinn fixing solution were prepared according to conventional methods.

(2) Fixation: the cysts were added into the Schaudinn fixing solution and fixed in a water bath at 40°C for 5 min or at room temperature for 20-30 min; and after centrifuging was carried out for 5 min (at 1,500 rpm), the supernatant was removed.

③ 2% iodine was added for acting for 30 min to remove mercury, and centrifuging was carried out to remove the supernatant.

④ 70% alcohol was added and stood for 1 h or overnight so as to remove mercury iodide; centrifuging was carried out to remove the supernatant; water was added and stood for 10 min; and centrifuging was carried out to remove the supernatant.

⑤ Mordanting: 4% iron alum solution was added; mordanting was carried out in a water bath at 40°C for 5 min or at room temperature for 30 min; centrifuging was carried out to remove the supernatant; and water was added to wash 3 times.

⑥ Staining: after centrifuging was carried out to remove the supernatant, 0.5% hematoxylin staining solution was added for staining in a water bath at 40°C for 5-15 min; centrifuging was carried out to remove the supernatant; and water was added to wash 3 times.

⑦ Color separation: after centrifuging was carried out to remove the supernatant, 2% iron alum solution was added for color separation; the fading conditions of the cysts were observed under a microscope while carrying out color separation until the internal structures of the cysts were clear, generally 3-5 min; and water was added to wash 3 times.

⑧ After centrifuging was carried out to remove the supernatant, water was added for soaking for 1 h (preferably overnight) so as to remove a color separation agent, and centrifuging was carried out to remove the supernatant.

⑨ Dehydrating: 50-95% alcohol and pure alcohol were gradually added for dehydrating; the dehydrating was carried out at each stage for 10 min; and centrifuging was carried out to remove the supernatant.

⑩ Transparentizing: 10 parts of a mixed solution with half pure alcohol and half ilex oil (or xylene) was added, and centrifuging was carried out to remove the supernatant; and 5 parts of the pure ilex oil or xylene solution was added, and centrifuging was carried out to remove the supernatant.

⑪ Mounting: a few drops of the ilex oil or xylene solution (depends on the amount of the precipitate) was added and uniformly mixed; 1/2 of the cyst suspension liquid was sucked by a small suction tube and dropped on a glass slide and then was uniformly stirred with 1 drop of a neutral gel, and then a coverslip was used for covering; and the product was naturally dried by air.

**In-vivo observation of different treatments on Acanthamoeba castellanii.**

50 mL of Acanthamoeba castellanii culture solutions under different treatments was washed with PBS 3 times, and the suspension was adjusted with PBS until 5 ul of PBS contained $1\times10^5$ Acanthamoeba castellanii trophozoites. An SPF-grade 6-week-old female BALB/c mouse was selected; and after intraperitoneal injection of the mouse, the survival rates of Acanthamoeba castellanii trophozoites and the cysts under different treatment groups were calculated respectively. If the experimental results proved that the corneal contact lens electrophoresis dissociation protein removal and sterilization instrument had killing and inhibiting effects on Acanthamoeba castellanii, the experiment would be popularized to animal model rabbits; and related experiments would be further carried out on rabbit cornea.

The contact lens cleaner of the present disclosure realizes the purpose of removing protein by electrophoresis dissociation; the protein electrophoresis principle in biomedicine is combined with the electrolysis technology, which is different from the conventional electrophoresis and electrolysis technology. The originality is that: the lacrimal proteins are charged under a weak alkaline condition; and in the electric field, the charged lacrimal proteins are adsorbed by the electrophoresis dissociation probes. According to the Embodiments and the Verification Embodiments 1-3, the technology can achieve an almost 100% protein removal effect; the self-developed NaCl cleaning solution having a specific pH value has dual energy efficiency of electrophoresis and electrolysis; the charged proteins are adsorbed by electrophoresis; CIO- having an appropriate concentration is generated in the electrolysis process and can decompose the stubborn deposited protein; and almost 100% deep protein cleaning and 99.9999% sterilization effects are achieved.

According to the present disclosure, protein is removed by the technical solution of combining electrophoresis and electrolytic dissociation. The cleaning process and the cleaning result are demonstrated in principle, so that the effective protein removal can be proved. Moreover, the cleaning effect is verified in a laboratory according to the principle, and an experimental result of 100% protein removal effect is also obtained. Therefore, the high-cleanliness cleaning method can protect the human eyes of a user from being infected by bacteria.

According to the present disclosure, the electrophoresis technology is utilized to separate the lacrimal proteins from the lens, and is cooperated with the electrolysis technology to hydrolyze the peptide bonds in the lacrimal proteins (protein undergoes irreversible hydrolysis under acidic or alkaline conditions); and the proteins can be hydrolyzed to form small molecules which are easily dissolved in water, thereby completely removing lacrimal proteins molecules and achieving the sterilization and disinfection effects.

According to the present disclosure, the 3N corneal contact lens electrophoresis dissociation protein removal and sterilization instrument uses the globally unique electrophoresis dissociation protein removing principle independently researched by 3N TECH. Its working mechanism is that a circuit switch is controlled by the contact button; the probes receive voltage difference after the solution containing NaCl is added into the lens cleaning tank of the cleaning bin, thus electrophoresis dissociation is realized, and further, protein removal and sterilization work on the corneal contact lens is realized. The electrophoresis dissociation protein removal technology is formed by superposing the biological electrophoresis technology and the NaCl electrolysis technology. The electrophoresis refers to a phenomenon that charged particles or molecules move in the electric field. Macromolecular protein, polypeptide, virus particles, even cells or micromolecular amino acid, nucleoside and the like of can perform directional electrophoresis in the electric field. Under the action of a 3N corneal contact lens reduction instrument, the lacrimal proteins are charged and move towards the electrode under the action of the field intensity of the probe in the cleaning bin, so that the corneal contact lens is cleaned.

In conclusion, compared with a method for rubbing and soaking a lens in cooperation with a care solution in the prior art, the method for protein removal and sterilization by electrophoresis dissociation disclosed by the present disclosure has the advantages that the service life of the lens is prolonged; moreover, the cleaning effect can be quantified through a laboratory detection result under the support of the technical principle; and the cleanliness is high.

In the description of this specification, the description of the reference terms "one embodiment", "some embodiments", "an example", "a specific example", "some examples," and the like means that specific features, structures, materials or characteristics described in combination with the embodiment(s) or example(s) are included in at least one embodiment or example of the present disclosure. In this specification, schematic descriptions of the foregoing terms are not necessarily directed at the same embodiment or example. In addition, the described specific features, structures, materials, or characteristics may be combined in a proper manner in any one or more of the embodiments or examples. In addition, a person in the art may combine different embodiments or examples described in this specification.

Although the embodiments of the present disclosure have been shown and described above, it can be understood that, the foregoing embodiments are exemplary and should not be understood as limitation to the present disclosure. A person of ordinary skill in the art can make changes, modifications, replacements, or variations to the foregoing embodiments within the scope of the present disclosure.

## Claims

1. A method for protein removal and sterilization by electrophoresis dissociation, comprising:

   providing a contact lens cleaner comprising a lens cleaning tank having two electrophoresis dissociation probes arranged opposite each other therein;
   adding a lens to be cleaned and an electrolyte solution serving as a cleaning solution into the lens cleaning tank, the electrolyte solution comprising a solution containing $Cl^-$, and forming an anode and a cathode by the two electrophoresis dissociation probes under a circuit loop;
   enabling lacrimal proteins attached to a surface of the lens to be cleaned to be charged in the solution containing $Cl^-$, and enabling the charged lacrimal proteins to move towards a position of one electrode opposite to an electrical property thereof;
   enabling the $Cl^-$ in the electrolyte solution to move towards the anode, and to lose electrons and be oxidized into chlorine which is dissolved in the electrolyte solution to generate hypochlorite; and the hypochlorite reacting with the lacrimal proteins in an oxidation reaction in the lens cleaning tank to degrade the lacrimal proteins.

2. The method for protein removal and sterilization by electrophoresis dissociation according to claim 1, wherein

   the hypochlorite is a strong oxidant, and undergoes a self-redox reaction in the electrolyte solution so as to be decomposed to generate hydrogen ions, chloride ions and oxygen; and the hypochlorite can absorb electrons of functional proteins on cell walls of microorganisms in the lens cleaning tank during decomposition, so that the functional proteins on the cell walls of the microorganisms are inactivated, and the microorganisms are killed; and/or,
   the hypochlorite absorbs electrons of functional proteins on shells of the microorganisms in the lens cleaning tank during decomposition, so that the functional proteins on the cell walls of the microorganisms are inactivated, and the microorganisms are killed; and/or,
   the hypochlorite permeates into cells of the microorganisms and undergoes an oxidation reaction with mycoprotein, nucleic acid and enzymes in the cells of the microorganisms, so that the microorganisms are killed.

3. The method for protein removal and sterilization by electrophoresis dissociation according to claim 2, wherein the microorganisms comprise bacteria and viruses;

   the hypochlorite absorbs the electrons of the functional proteins on the cell walls of the bacteria during decomposition, and thus the bacteria are inactivated; and
the hypochlorite absorbs the electrons of the functional protein on the shells of the viruses during decomposition, and thus the viruses are inactivated.

4. The method for protein removal and sterilization by electrophoresis dissociation according to claim 1, wherein the hypochlorite reacting with the lacrimal proteins in an oxidation reaction in the lens cleaning tank to degrade the lacrimal proteins specifically comprises:

the hypochlorite reacts with a peptide chain forming a lacrimal protein framework to generate chloral formamide; a peptide chain formed by chloral formamide is hydrolyzed in the solution; and peptide bonds in the peptide chain are broken and decomposed into small molecular protein and amino acid, and thus the lacrimal proteins are degraded; and/or
the hypochlorite reacts with a side chain of the peptide chain forming the lacrimal protein framework; the side chain comprises a lysine side chain which reacts with the hypochlorite, and chloramine is formed on the lysine side chain of the lacrimal proteins and amino of the lacrimal proteins; the chloramine is decomposed to form organic molecular fragments [1, 2] in a carbonyl form; and the peptide bonds on the lysine side chain are broken and decomposed into small molecular protein and amino acid, and thus the lacrimal proteins are degraded.

5. The method for protein removal and sterilization by electrophoresis dissociation according to claim 4, wherein the hypochlorite reacts with the side chain of the peptide chain forming the lacrimal protein framework; the side chain further comprises one or more of a methionine side chain, a cysteine side chain, a histidine side chain, an $\alpha$-amino acid side chain, a tryptophan side chain, a tyrosine side chain, an arginine side chain and a glutamic acid side chain; and
a reaction activity of the lysine side chain and the hypochlorite is between the reaction activity of the tryptophan side chain and the reaction activity of the tyrosine side chain.

6. The method for protein removal and sterilization by electrophoresis dissociation according to claim 1, wherein a circuit power supply of the contact lens cleaner is switched on, then the two electrophoresis dissociation probes and the electrolyte solution form a conductive loop to generate current, and the two electrophoresis dissociation probes respectively form the anode and the cathode; and

the charged lacrimal proteins in the electrolyte solution move to the electrode opposite to the electrical property thereof;
positively charged hydrogen ions in the solution containing Cl$^-$ move to the cathode and undergo a reduction reaction near the cathode so as to generate hydrogen; and
negatively charged chloride ions in the solution containing Cl$^-$ move to the anode in the solution and undergo an oxidation reaction to generate chlorine; chlorine reacts with water in the solution to generate hypochlorite; and the hypochlorite enables the lacrimal proteins to be degraded from the lens to be cleaned.

7. The method for protein removal and sterilization by electrophoresis dissociation according to claim 1, wherein the solution containing Cl$^-$ comprises a solution containing Cl- with a concentration of 0.01-10%; and
the solution containing Cl$^-$ comprises one or more of a NaCl solution, a KCl solution, a MgCl$_2$ solution and a lens care solution containing Cl-.

8. The method for protein removal and sterilization by electrophoresis dissociation according to claim 7, wherein the solution containing Cl$^-$ comprises a NaCl solution having a concentration of 0.9%.

9. A method for protein removal and sterilization by electrophoresis dissociation, comprising:

providing a contact lens cleaner comprising a lens cleaning tank having at least two electrophoresis dissociation probes arranged opposite each other therein;
adding a lens to be cleaned and an electrolyte solution serving as a cleaning solution into the lens cleaning tank;
forming two electrodes having opposite electrical properties by the two electrophoresis dissociation probes under a circuit loop, lacrimal proteins on the lens to be cleaned being charged in the electrolyte solution, and the charged lacrimal proteins moving towards the electrode opposite to the electrical property thereof; and
enabling the electrolyte solution to react in the lens cleaning tank to generate a strong oxidant which undergoes an oxidation reaction with the lacrimal proteins, thereby degrading the lacrimal proteins.

**10.** The method for protein removal and sterilization by electrophoresis dissociation according to claim 9, wherein the strong oxidant absorbs electrons of surface proteins on cell walls of microorganisms in the lens cleaning tank during decomposition, so that the surface proteins on the cell walls of the microorganisms are inactivated, and the microorganisms are killed; and

the electrolyte solution comprises an ionic solution which contains chlorate and can be used as a lens care solution.

**11.** A contact lens cleaner for implementing protein removal and sterilization by electrophoresis dissociation, comprising:

a main machine shell, comprising a cleaning bin, a base, a lens cleaning tank being arranged in the cleaning bin, an integrated circuit being arranged in the base, and an electrophoresis dissociation probe in the lens cleaning tank being electrically connected to the integrated circuit;
wherein the cleaning bin is a magnetic control integrated bin;
each electrophoresis dissociation probe is an electrophoresis dissociation probe having a nano-scale coating material; the electrophoresis dissociation probe extends into the lens cleaning tank from a bottom of the lens cleaning tank, and the electrophoresis dissociation probe is close to an inner wall of the lens cleaning tank; and
a top end of the electrophoresis dissociation probe is lower than an upper edge of a tank wall of the lens cleaning tank.

**12.** The contact lens cleaner according to claim 11, wherein the integrated circuit comprises a power supply management circuit, a booster circuit and a switch; and the switch comprises a touch button arranged on the cleaning bin or the base; starting an electrophoresis dissociation protein removal function of the cleaner specifically comprises:

manually touching the touch button to switch on the power supply management circuit to form a conductive loop and generate a loop current, a voltage difference being formed by the loop current through the booster circuit; receiving the voltage difference by two electrophoresis dissociation probes in the cleaning tank; and forming an electrolytic tank in the lens cleaning tank through the electrolyte solution in the lens cleaning tank.

**13.** The contact lens cleaner according to claim 12, wherein the power supply management circuit comprises a power supply; the power supply provides electric energy for the conductive loop; the two electrophoresis dissociation probes form an anode and a cathode in the lens cleaning tank after being electrified; lacrimal proteins on the lens move towards a position of the electrode opposite to the electrical property thereof in the electrolyte solution in the lens cleaning tank; and the electrolyte solution reacts in the lens cleaning tank to generate ions which react with the lacrimal proteins.

Fig.1

Fig. 2

Fig. 3

Fig. 4

Lens(a)  Lens(b)

Fig. 5

Lens(a1)  Lens(b1)

Fig. 6

## INTERNATIONAL SEARCH REPORT

| International application No. |
| --- |
| **PCT/CN2021/109100** |

**A.  CLASSIFICATION OF SUBJECT MATTER**

G02C 13/00(2006.01)i;  A61L 12/02(2006.01)i;  A61L 12/10(2006.01)i

According to International Patent Classification (IPC) or to both national classification and IPC

**B.  FIELDS SEARCHED**

Minimum documentation searched (classification system followed by classification symbols)

G02C 13, A61L 12; CPC: G02C 13/008, A61L 12/023, A61L 12/107

Documentation searched other than minimum documentation to the extent that such documents are included in the fields searched

Electronic data base consulted during the international search (name of data base and, where practicable, search terms used)

CNABS; CNTXT; WPABSC; USTXTC; ENTXTC; VEN; OETXT; 百度: 三个臭皮匠, 孙碧霞, 隐形眼镜, 塑形镜, 接触镜, OK镜, 电泳, 电解, 次氯酸, 次卤酸, contact, lens, orthokeratology, electrophoretic, electrolytic, hypochlorous, hypohalogenous

**C.  DOCUMENTS CONSIDERED TO BE RELEVANT**

| Category* | Citation of document, with indication, where appropriate, of the relevant passages | Relevant to claim No. |
| --- | --- | --- |
| X | 南京热线 (Nanjing Rexian). "自动清洗, 随身便携, 用它每天都戴新镜片, 一年帮你省下上千块! (Non-official translation: 3N Contact Lens Cleaner: Automatic and Portable, Better Quality and Less Cost!)" *https://www.163.com/dy/article/F15RGB1H0545AWG3.html*, 22 July 2020 (2020-07-22), pp. 3-9 | 1-13 |
| Y | CN 209070255 U (SUZHOU SANGECHOUPIJIANG BIOLOGICAL TECHNOLOGY CO., LTD.) 05 July 2019 (2019-07-05) description, paragraphs 0048-0061, and figures 1-10 | 1-13 |
| Y | US 2017173206 A1 (NOVARTIS AG.) 22 June 2017 (2017-06-22) description paragraphs 0026-0079, figures 2A-2C | 1-13 |
| Y | US 4732185 A (BAREMEK PTY LIMITED) 22 March 1988 (1988-03-22) description column 1 line 35 to column 4 line 54, figures 1-3 | 1-13 |
| Y | WO 2014042407 A1 (DOLKI KOREA LTD et al.) 20 March 2014 (2014-03-20) description column 1 line 35 to column 4 line 54, figures 1-3 | 1-13 |
| Y | US 5449442 A (TOMEY TECHN CORP) 12 September 1995 (1995-09-12) description, column 7, line 6 to column 10, line 52 | 1-13 |

☑ Further documents are listed in the continuation of Box C.      ☑ See patent family annex.

| | |
| --- | --- |
| *      Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **29 October 2021** | **08 November 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

| International application No. |
| --- |
| **PCT/CN2021/109100** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 5252291 A (CIBA-GEIGY CORPORATION) 12 October 1993 (1993-10-12)<br>    entire document | 1-13 |
| A | GB 2072371 A (TOYO CONTACT LENS CO LTD) 30 September 1981 (1981-09-30)<br>    entire document | 1-13 |

Form PCT/ISA/210 (second sheet) (January 2015)

**INTERNATIONAL SEARCH REPORT**
Information on patent family members

International application No.

**PCT/CN2021/109100**

| Patent document cited in search report | | | Publication date (day/month/year) | Patent family member(s) | | | Publication date (day/month/year) |
|---|---|---|---|---|---|---|---|
| CN | 209070255 | U | 05 July 2019 | CN | 110794593 | A | 14 February 2020 |
| US | 2017173206 | A1 | 22 June 2017 | EP | 3125957 | A1 | 08 February 2017 |
| | | | | CA | 2939880 | A1 | 08 October 2015 |
| | | | | CA | 2939880 | C | 22 May 2018 |
| | | | | US | 10525158 | B2 | 07 January 2020 |
| | | | | WO | 2015153161 | A1 | 08 October 2015 |
| | | | | JP | 2017510354 | A | 13 April 2017 |
| | | | | JP | 6273039 | B2 | 31 January 2018 |
| US | 4732185 | A | 22 March 1988 | AU | 4273085 | A | 19 September 1985 |
| | | | | AU | 565948 | B2 | 01 October 1987 |
| | | | | CA | 1243467 | A | 25 October 1988 |
| | | | | US | 4921544 | A | 01 May 1990 |
| WO | 2014042407 | A1 | 20 March 2014 | KR | 20140034101 | A | 19 March 2014 |
| US | 5449442 | A | 12 September 1995 | JP | H07104221 | A | 21 April 1995 |
| | | | | EP | 0648504 | A2 | 19 April 1995 |
| | | | | EP | 0648504 | A3 | 14 February 1996 |
| US | 5252291 | A | 12 October 1993 | NO | 932450 | D0 | 06 July 1993 |
| | | | | JP | H06230326 | A | 19 August 1994 |
| | | | | MX | 9303928 | A | 31 January 1994 |
| | | | | EP | 0578612 | A1 | 12 January 1994 |
| | | | | NZ | 248070 | A | 27 February 1996 |
| | | | | FI | 933085 | A0 | 05 July 1993 |
| | | | | FI | 933085 | A | 08 January 1994 |
| | | | | TW | 204398 | B | 21 April 1993 |
| | | | | AU | 4179793 | A | 13 January 1994 |
| | | | | AU | 666376 | B2 | 08 February 1996 |
| | | | | CA | 2099824 | A1 | 08 January 1994 |
| | | | | IL | 106209 | D0 | 15 November 1993 |
| | | | | ZA | 9304835 | B | 07 January 1994 |
| | | | | NO | 932450 | A | 10 January 1994 |
| | | | | ZA | 934835 | A | 23 February 1994 |
| GB | 2072371 | A | 30 September 1981 | JP | S56119113 | A | 18 September 1981 |
| | | | | JP | S6027965 | B2 | 02 July 1985 |
| | | | | GB | 2072371 | B | 11 July 1984 |
| | | | | CA | 1163906 | A | 20 March 1984 |
| | | | | FR | 2479491 | A1 | 02 October 1981 |
| | | | | FR | 2479491 | B1 | 08 February 1985 |
| | | | | DE | 3106290 | A1 | 14 January 1982 |
| | | | | DE | 3106290 | C2 | 07 March 1985 |

Form PCT/ISA/210 (patent family annex) (January 2015)